(19) Europäisches Patentamt
European Patent Office
Office européen des brevets

(11) Publication number: **0 151 886**
**A1**

(12) # EUROPEAN PATENT APPLICATION

(21) Application number: 84309071.3

(22) Date of filing: 21.12.84

(51) Int. Cl.[4]: C 07 C 69/14
C 07 C 67/40

(30) Priority: 12.01.84 GB 8400785

(43) Date of publication of application: 21.08.85 Bulletin 85/34

(84) Designated Contracting States: DE FR GB IT

(71) Applicant: DAVY McKEE (LONDON) LIMITED
250, Euston Road
London NW1 2PG(GB)

(72) Inventor: Kippax, John Wilson
John Clapham House Firby
Bedale North Yorkshire DL8 2PW(GB)

(72) Inventor: Turner, Keith
12 The Avenue Fairfield
Stockton-on-Tees Cleveland(GB)

(72) Inventor: Harris, Norman
22 Grantham Road
Norton Cleveland(GB)

(72) Inventor: Sharif, Mohammad
19 Maidstone Drive Marton
Middlesbrough Cleveland(GB)

(74) Representative: Eyles, Christopher Thomas et al,
BATCHELLOR, KIRK & EYLES 2 Pear Tree Court
Farringdon Road
London, EC1R 0DS(GB)

(54) **Process for the preparation of esters.**

(57) A process for the production of a primary $C_{2+}$ alkyl ester of an alkyl carboxylic acid comprises contacting a vaporous mixture containing a primary $C_{2+}$ alkanol and hydrogen in an alkanol:hydrogen molar ratio of from about 1:10 to about 1000:1 at a combined partial pressure of alkanol and hydrogen of from about 0.1 bar up to about 40 bar and at a temperature in the range of from about 180°C to about 300°C in a catalytic reaction zone with a catalyst consisting essentially of a reduced mixture of copper oxide and zinc oxide, and recovering a reaction product mixture containing a primary $C_{2+}$ alkyl ester of an alkyl carboxylic acid, which ester contains twice as many carbon atoms as the primary $C_{2+}$ alkanol.

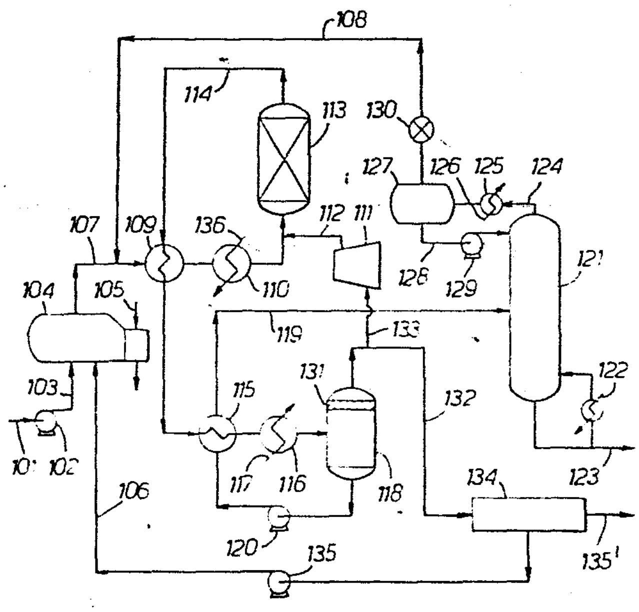


FIG. 2.

Croydon Printing Company Ltd

# PROCESS

This invention relates to a process for the production of esters, more particularly to a process for the production of a primary $C_{2+}$ alkyl ester of an alkyl carboxylic acid from a primary $C_{2+}$ alkanol.

Esters, such as ethyl acetate, are relatively expensive bulk chemicals. Typically they are produced by esterification of the carboxylic acid with the corresponding alcohol, e.g.:

$CH_3.CO.OH + CH_3.CH_2.OH = CH_3.CO.O.CH_2.CH_3 + H_2O$.

In some cases the Tischenko condensation reaction can be used. For example ethyl acetate can be produced from acetaldehyde:

$2\ CH_3.CHO \quad = \quad CH_3.CO.O.CH_2.CH_3$.

Sometimes esters are by-products of other reactions. Thus some ethyl acetate is produced as a by-product in the liquid phase oxidation of n-butane.

Ethanol may be available in large quantity, either as a product of hydration of ethylene or, in certain countries, as a fermentation product. Acetic acid is typically produced by oxidation of n-butane or other hydrocarbons, such as naphtha. The production of acetic acid by the carbonylation of methanol using cobalt, or more recently rhodium, catalysts has also had wide industrial application. In certain circumstances ethanol may be available in excess capacity, whilst acetic acid is not available.

The production of esters directly from primary alcohols has been described on various occasions in the literature. For example, dehydrogenation of methanol over Cu-Zr-Zn catalysts at temperatures of 100 to 400°C to yield methyl formate has been described in Belgian Patent Specification No. 879915 (see also Chemical Abstacts, Vol 93 (1980), No.

204080f at page 635). In this process the Cu-Zr-Zn ratio is 1:0.01-2.0:0.01-2.

French Patent Specification No. 1000505 (see Chemical Abstracts, Vol 51 (1957), 3061b) describes passage of methanol vapour over an Al-Cu catalyst at 180 to 250°C to form methyl formate by dehydrogenation.

Catalytic dehydrogenation of alcohols with reduced copper under ultraviolet light was described by S. Nakamura et al, in Bulletin of the Chemical Society of Japan, Vol. 44, pages 1072 to 1078 (1971).

K. Takeshita et al described reduced copper catalysed conversion of primary alcohols into esters and ketones in Bulletin of the Chemical Society of Japan, Vol 51(9), pages 2622 to 2627 (1978). These authors mention that the mechanism for ester formation has been described in the literature as the Tischenko reaction.

None of these prior disclosures has resulted in adoption of the dehydrogenation of an alkanol as a commercial method of producing esters.

It would accordingly be desirable to find a commercially viable method of upgrading ethanol to a more valuable product, particularly where there is an overcapacity for ethanol. It would also be desirable to provide a novel route to ethyl acetate and other esters which obviates the need for expensive capital plant, such as a separate acetic acid plant.

The present invention accordingly seeks to provide a novel process for production of $C_{2+}$ alkyl esters of alkyl carboxylic acids from $C_{2+}$ primary alcohols, enabling production of such esters at relatively low cost and involving simple plant.

According to the present invention there is provided a process for the production of a primary

$C_{2+}$ alkyl ester of an alkyl carboxylic acid which comprises contacting a vaporous mixture containing a $C_{2+}$ primary alkanol and hydrogen in an alkanol:hydrogen molar ratio of from about 1:10 to about 1000:1 at a combined partial pressure of alkanol and hydrogen of from about 0.1 bar up to about 40 bar and at a temperature in the range of from about 180°C to about 300°C in a catalytic reaction zone with a catalyst consisting essentially of a reduced mixture of copper oxide and zinc oxide, and recovering a reaction product mixture containing a primary $C_{2+}$ alkyl ester of an alkyl carboxylic acid, which ester contains twice as many carbon atoms as the primary $C_{2+}$ alkanol.

Preferably the primary $C_{2+}$ alkanol contains from 2 to about 8 carbon atoms. In a particularly preferred process the primary $C_{2+}$ alkanol is ethanol and the ester is ethyl acetate. Other primary $C_{2+}$ alkanols which can be mentioned include n-propanol, n-butanol, n-pentanol, n-hexanol, and 2-ethylhexanol; these yield respectively n-propyl propionate, n-butyl butyrate, n-pentyl valerate, n-hexyl caproate, and 2-ethylhexyl 2-ethylhexanoate.

The vaporous mixture supplied to the catalytic reaction zone contains, in addition to the primary $C_{2+}$ alkanol (e.g. ethanol), hydrogen either alone or in admixture with other gases (desirably gases inert to the ester and the catalyst). Examples of such other gases include inert gases such as nitrogen, methane, and argon. The vaporous mixture may further comprise a minor amount, for example from about 0.1 mole up to about 10 moles or more, per 100 moles of primary $C_{2+}$ alkanol, of the corresponding alkanal. It may also include a minor amount of recycled ester product.

The process of the present invention is conducted at a temperature of between about 180°C and about 300°C, preferably at a temperature of at least about 200°C. Typically, the temperature is between about 240°C and about 280°C.

The combined partial pressure of primary $C_{2+}$ alkanol and hydrogen lies in the range of from about 0.1 bar to about 40 bar, preferably in the range of from about 1 bar up to about 35 bar. The primary $C_{2+}$ alkanol:hydrogen ratio in the vaporous mixture fed into contact with the catalyst may be from about 1:10 to about 1000:1. Usually it will not exceed about 400:1 or about 500:1 and may be no more than about 120:1. The total pressure (i.e. the sum of the partial pressure of hydrogen, primary $C_{2+}$ alkanol, ester, inert gas (if any) and any by-products) preferably does not exceed about 100 bar and even more preferably does not exceed about 50 bar.

Although we do not wish to be bound by the accuracy or otherwise of the following theoretical explanation, it would appear from our experimental work that the reaction proceeds in two steps. The first step involves dehydrogenation of the primary $C_{2+}$ alkanol to yield the corresponding alkanal which then can react in a second step with further alkanal or with primary $C_{2+}$ alkanol to form the ester and release hydrogen. For example, when treating ethanol by the process of the invention, we think that the reactions involved are:

1 $CH_3.CH_2.OH = CH_3.CHO + H_2$

2 (a) $CH_3.CH_2.OH + CH_3.CHO = CH_3.CO.O.CH_2.CH_3 + H_2$

or:

(b) $2\ CH_3.CHO = CH_3.CO.O.CH_2.CH_3$.

This can be summarised as the following overall reaction:

$$2\ CH_3.CH_2.OH = CH_3.CO.O.CH_2.CH_3 + 2H_2.$$

In the course of our experiments we have found that the product mixture often contains amounts of the alkanal corresponding to the starting primary $C_{2+}$ alkanol. As this alkanal is postulated to be an intermediate in the process of the invention it can be ignored in calculating the overall selectivity of conversion of the primary $C_{2+}$ alkanol to product ester.

Besides the alkanal the product mixture may include small amounts of other by-products, including higher alcohols, ketones, carbon oxides, methane and other alkanes. For example, when treating ethanol by the process of the invention, we have identified in the product mixture traces of the following minor by-products: iso-propanol, diethyl ether, methyl ethyl ketone, n-butanol, acetone, carbon monoxide, carbon dioxide, methane and ethane.

The catalyst is a mixed metal oxide catalyst that consists essentially of a reduced mixture of copper oxide and zinc oxide. By the term "consists essentially of" we mean that the catalyst includes as essential ingredients in the mixture before reduction copper oxide and zinc oxide, but may also include amounts of other metal oxides that do not materially alter the basic characteristics of the catalyst as well as inert fillers or supports, such as carbon. Such characteristics include the ability to catalyse production from $C_{2+}$ alkanols of $C_{2+}$ alkyl esters of alkyl carboxylic acids, in the presence of hydrogen at moderate temperatures, for example in the range of from about 180°C to about 300°C, and low total pressures, for example in the range of from about 5 bar to about 100 bar, with high selectivity to the desired esters, typically in excess of 90% (if any

alkanal formed is disregarded), and with a marked absence of significant amounts of other by-products, typically less than 10% in total of by-products.

The catalyst may be derived from a mixture which contains only copper oxide and zinc oxide. Alternatively it may include one or more other materials, such as an inert support or other material that is effectively catalytically inactive in the ester formation reaction.

Hence the catalyst may be derived from a mixture of CuO and ZnO, which before reduction contains from about 5 to about 95% by weight, preferably from about 20 to about 85% by weight, and typically from about 25 to about 70% by weight, of CuO and from about 95 to about 5% by weight, preferably from about 80 to about 15% by weight, and typically from about 75 to about 30% by weight, of ZnO. Hence the mixture may contain, for example, from about 20 to about 40% by weight of CuO and from about 60 to about 80% by weight of ZnO. A preferred mixture, for example contains from about 30 to about 36% by weight of CuO and from about 62 to about 68% by weight of ZnO. Other particularly preferred mixtures contain from about 65 to about 85% by weight of CuO and from about 35 to about 15% by weight of ZnO, for example mixtures containing from about 60 to about 75% (e.g. about 68 to about 75%) by weight of CuO and from about 40 to about 25% (e.g. about 32 to about 25%) by weight of ZnO.

As already mentioned the catalyst may contain minor amounts of at least one carrier material such as carbon, titanium oxide, zirconium oxide, manganese dioxide, silica, diatomaceous earth, kieselguhr, or aluminium oxide. Such other materials

do not usually comprise in total more than about 20% by weight of the catalyst. The presence of minor amounts of other materials can be tolerated in the mixture from which the catalyst is derived, provided that these do not significantly affect the catalyst performance. For example, it is known that basic oxides such as magnesium oxide or barium oxide tend to promote alcohol condensation and dehydration reactions. Hence incorporation of excessive quantities of alkaline oxides in the mixture to be reduced will tend to promote "heavies" formation and to produce one or more alkenes as by-products. For example, in the production of ethyl acetate from ethanol, inclusion of increasing amounts of basic oxides in the mixture from which the catalyst is derived will tend to produce increasing amounts of n-butanol and ethylene, particularly at the higher end of the temperature range. For this reason, in the case of sodium it is best not to exceed about 0.5% by weight, calculated as oxide. For other less basic oxides, such as magnesium oxide or barium oxide, higher amounts may be tolerated in the oxide mixture, for example up to about 5% by weight or more. Preferably, however, the total content of basic oxides is no more than about 1% by weight of the catalyst.

Other preferred catalysts include mixtures containing from about 40 to about 50 weight percent each of CuO and ZnO and from 0 to about 20 weight percent, typically from about 5 to about 20 weight percent, of alumina. Hence preferred catalysts of this type comprise from about 40 to about 47.5 weight percent each of ZnO and CuO and from about 5 to about 20 weight percent of alumina.

The catalyst is, however, preferably essentially free from other metals, particularly from

metals of Group VIII of the Periodic Table, such as Fe, Co, Ni, Ru, Rh, Pd, Os, Ir, and Pt, as well as from Group VIB metals such as Cr, Mo, and W, from the metals Ag, Re, Au and Cd, and also from elements of atomic number 80 and above, e.g. Hg and Pb. By the term "essentially free" we mean that the catalyst contains not more than about 0.1 wt% (i.e. not more than about 1000 ppm), and preferably not more than about 250 ppm, of the element in question. Such metals include metals which, under the reaction conditions used in the process of the invention can cause side reactions or which lack catalytic activity or are catalyst poisons.

The catalyst may be prepared by any of the methods known in the art of forming a composite of copper oxide and zinc oxide. The catalyst may be prepared by mixing the separate oxides, by coprecipitation of the oxalates, nitrates, carbonates, or acetates, followed by calcination. The coprecipitation method is preferred. Generally, the mixture of CuO and ZnO is reduced by hydrogen or carbon monoxide as reducing gas, optionally in admixture with an inert gas such as nitrogen or argon, at a temperature in the range of between about 160°C and about 250°C for several hours, preferably for 8 to 24 hours, prior to contact with the vaporous mixture containing primary $C_{2+}$ alkanol and hydrogen. If the catalyst is charged in a pre-reduced form the period required for reduction can be reduced accordingly.

The mixture of CuO and ZnO is reduced prior to its use as catalyst. Hydrogen or CO, or mixtures thereof, are generally mixed with a diluent gas such as steam, nitrogen, or combustion gas, to maintain the catalyst bed temperature and to carry away the heat of

reduction.

Reduction of the mixture of CuO and ZnO is complete when no more hydrogen is being reacted as shown by analysis of the inlet and outlet hydrogen. Complete reduction of the mixture occurs when the total amount of water produced in the reduction is equal to the stoichiometric value of water which should be produced when a given amount of copper oxide is reduced to copper. This value is about 0.079 kg of water per kg of catalyst for a mixture containing 35 weight percent of CuO.

An inert carrier material may be included in the CuO-ZnO catalyst composition. The catalyst is generally formed into pellets, tablets, or any other suitable shape prior to use, by conventional techniques.

It is advantageous that the mixture of CuO and ZnO have an internal surface area of from about 25 to about 50 sq.m per gram. The internal surface area may be determined by the well-known BET method.

The process of the present invention is most conveniently carried out in a continuous manner, although semi-continuous or batch operations may also be employed. In the preferred method of continuous operation, a primary $C_{2+}$ alkanol, hydrogen, and, optionally, a carrier gas such as nitrogen or a mixture of nitrogen with methane and/or other inert gases, may be brought together and, under the desired pressure, contacted in the vaporous state with the catalyst. The reaction zone can be, for example, an elongated tubular reactor filled with the catalyst. Alternatively it can be an adiabatic reactor. The reaction is endothermic. Hence, if adiabatic operation is used, an appropriately high inlet temperature should be selected in order to avoid

premature quenching of the reaction due to the endothermic nature of the process. Several reactors in series can be used.

The reaction mixture from the catalytic reaction zone contains, besides hydrogen (and possibly also other gaseous components such as nitrogen, carbon oxides, methane and ethane), unreacted primary $C_{2+}$ alkanol, ester and possibly also byproducts, such as the alkanal corresponding to the primary $C_{2+}$ alkanol. The condensible components, including primary $C_{2+}$ alkanol, $C_{2+}$ alkanal and product ester, can be condensed and separated in any convenient manner, e.g. distillation in one or more stages under normal, elevated or reduced pressure. Unreacted primary $C_{2+}$ alkanol can be recycled to the catalytic reaction zone, as can also gaseous components and any byproduct $C_{2+}$ alkanal. In some cases the primary $C_{2+}$ alkanol may form an azeotrope with the product ester. Such azeotropes can be separated from the bulk of the product ester as an overhead product by distillation under normal pressure, or under superatmospheric pressure if operation under superatmospheric pressure reduces the concentration of ester in the azeotrope. Any azeotrope thus separated can be used as fuel or recycled for admixture into the vaporous mixture to be contacted with the catalyst. In accordance with conventional practice purge streams can be taken in order to control the level of inerts and/or byproducts in the recycled streams.

According to one preferred procedure the reaction product mixture is distilled at elevated pressure, for example at a pressure of from about 10 bar to about 40 bar, to yield an overhead product stream comprising an azeotrope of the primary $C_{2+}$

alkanol and the product ester and a bottom product stream comprising predominantly said ester. Conveniently material of the overhead product stream is recycled for admixture with primary $C_{2+}$ alkanol feed.

In an alternative preferred procedure the reaction product mixture is distilled in a plurality of stages, including a low pressure distillation stage at a pressure of not more than about 5 bar and a subsequent high pressure distillation stage at a pressure of from about 10 bar to about 40 bar, said low pressure distillation stage resulting in production of a bottom product which comprises predominantly primary $C_{2+}$ alkanol and an overhead product which is a first azeotrope of the primary $C_{2+}$ alkanol and a major molar amount of said ester, the first azeotrope being thereafter subjected to distillation in said high pressure distillation stage to produce a bottom product which comprises predominantly said ester and an overhead product which is a second azeotrope of a major molar amount of the primary $C_{2+}$ alkanol and said ester, said second azeotrope being conveniently recycled to said low pressure distillation stage. When the product ester and the primary $C_{2+}$ alkanol form an azeotrope the reaction product mixture can be distilled essentially at reaction pressure, prior to said low pressure distillation stage, to remove alkanal by-product as an overhead product. It will usually be preferred to recycle said alkanal by-product for admixture with fresh primary $C_{2+}$ alkanol feed.

The ester product, e.g. ethyl acetate, can be used as such or can be reacted further. For example, the ester can be hydrolysed in conventional manner to liberate the alkyl carboxylic acid, e.g. acetic acid,

and release primary $C_{2+}$ alkanol, e.g. ethanol, for recycle (if desired) to the catalytic reaction zone. If the process of the invention is combined with hydrolysis in this way then the overall reaction becomes, for example:

$CH_3.CH_2OH + H_2O = CH_3.CO.OH + 2H_2$.

The process of the invention can be used to advantage in the treatment of methyl acetate produced as a byproduct of polyvinyl alcohol. Polyvinyl alcohol is produced by polymerisation of vinyl acetate, followed by transesterification of the resulting polyvinyl acetate with methanol which yields polyvinyl alcohol and methyl acetate. This latter compound can be hydrolysed to yield methanol, which can be recycled by the plant operator, and acetic acid. The acetic acid is of little value to the plant operator and it is often scarcely worthwhile to return the acetic acid to the manufacturer of vinyl acetate.

It would be desirable to provide a better method of treating such byproduct methyl acetate. We can now propose a two stage treatment which yields, in addition to the methanol required by the operator of the polyvinyl alcohol plant for transesterification of polyvinyl acetate, a product with a higher intrinsic value than acetic acid, viz. ethyl acetate. According to this proposal the byproduct methyl acetate is subjected to hydrogenolysis according to the teachings of International Patent Publication No. WO82/03854 to yield a mixture of methanol and ethanol, the former of which is recycled for further transesterification, whilst the ethanol is treated by the process of the invention to yield ethyl acetate. Both steps require the presence of hydrogen in the feed to the catalyst (although the production of ethyl acetate from ethanol results in net production of hydrogen) but the capital

cost of the plant involved is in each case relatively modest.

When operating continuously it is preferred to supply the primary $C_{2+}$ alkanol to the catalytic reaction zone at a liquid hourly space velocity of at least about 0.1 $hr^{-1}$, preferably in the range of from about 0.2 $hr^{-1}$ to about 1 $hr^{-1}$, e.g. about 0.5 $hr^{-1}$. Usually the liquid hourly space velocity will not exceed about 5.0 $hr^{-1}$.

The invention is further described in the following Examples.

Examples 1 to 3

The apparatus is illustrated diagrammatically in Figure 1 of the accompanying drawings. This was constructed out of stainless steel and was immersed in a fluidised sand bath (not shown) for heating purposes.

In operation a mixture of hydrogen and nitrogen was introduced by way of a pressure regulator and flow controller (not shown) through line 1 to the bottom end of a vaporiser 2 containing a number of steel balls 3. Ethanol was metered as a liquid to vaporiser 2 through line 4. The resulting vaporous mixture of ethanol, hydrogen and nitrogen was passed through preheating coil 5 to reactor 6. This contained a layer 7 of glass balls, on which rested the catalyst bed 8. The remainder of the reactor was filled with glass balls 9 and the upper end of the reactor was fitted with an exit tube 10 which led by way of a pressure let-down valve (not shown) to a condenser (not shown).

The exit gas flow rate was measured downstream from the condenser using a wet gas meter (also not shown).

At start up a charge of 50 ml of a granulated

copper oxide/zinc oxide catalyst (65 wt% CuO:35 wt% ZnO) was placed in the reactor which was then purged with nitrogen at 14.5 bar. A dilute $H_2$ in $N_2$ gaseous mixture at 14.5 bar was passed over the catalyst for 24 hours in order to effect catalyst reduction. The sand bath was raised to a temperature of 251°C.

Ethanol was then introduced into the vaporiser at the rate indicated in Table I below. The condensate was analysed by gas chromatography using a 1.82 metre long stainless steel column with an internal diameter of 3.18 mm containing 10% diethylene glycol succinate on Chromosorb PAW, a helium gas flow rate of 30 ml/minute and a flame ionisation detector. The instrument was fitted with a chart recorder having a peak integrator and was calibrated using a mixture of ethanol, acetaldehyde, ethyl acetate and water of known composition. The exit gas was also sampled and analysed by gas chromatography using the same technique. The identity of the peaks was confirmed by comparison of the retention times observed with those of authentic specimens of the materials in question. The following compounds were detected in the reaction mixture : ethanol, acetaldehyde, ethyl acetate and water. The results obtained are summarised in Table I. The heading "Liquid flow rate" refers to the ethanol flow rate to the vaporiser 2.

Table I

| Example No. | Liquid flow rate (ml/hr) | Gas flow rate (1/hr) | Gas composition (%$H_2$ in $N_2$) | Temperature (°C) | Ethanol Conversion (%) | Selectivity to ethyl acetate (%) |
|---|---|---|---|---|---|---|
| 1 | 10.3 | 52.9 | 0.77 | 251 | 51.1 | 87.1 |
| 2 | 16.6 | 54.2 | 6.47 | 250 | 45.2 | 80.6 |
| 3 | 8.7 | 51.1 | 8.36 | 239 | 35.5 | 94.9 |

Examples 4 to 6

Using a similar procedure to that described in Example 1 three further runs were carried out, all at 14.6 bar, using 50 ml of a different copper oxide/zinc oxide catalyst (35 wt% CuO:65 wt% ZnO). The results are listed in Table II.

Table II

| Example No. | Liquid flow rate (ml/hr) | Gas flow rate (l/hr) | Gas composition (% $H_2$ in $N_2$) | Temperature (°C) | Ethanol Conversion (%) | Selectivity to ethyl acetate (%) |
|---|---|---|---|---|---|---|
| 4 | 17.6 | 18.6 | 1.33 | 254 | 52.8 | 93.4 |
| 5 | 42.1 | 17.0 | 0.88 | 220 | 20.9 | 93.3 |
| 6 | 41.8 | 22.3 | 1.33 | 251 | 38.9 | 95.5 |

Example 7

Using the same catalyst as in Examples 4 to 6 and a similar procedure to that used in Examples 1 to 3, a solution of 2.5% w/v acetaldehyde in ethanol was used as liquid feed. At a liquid feed rate of 41.6 ml/hr a temperature of 251°C, a pressure of 14.6 bar and a gas flow rate of 21.6 l/hr of 1.33% $H_2$ in $N_2$, the observed conversion of ethanol was 37.8% with a selectivity of 91.4% to ethyl acetate.

In order that the invention may be clearly understood and readily carried into effect two preferred processes in accordance with the invention will now be described, by way of example only, with reference to Figures 2 and 3 of the accompanying drawings, each of which is a flow diagram of a plant designed for the production of ethyl acetate from ethanol.

It will be appreciated by those skilled in the art that Figures 2 and 3 are diagrammatic and that further items of equipment, such as temperature and pressure sensors, pressure relief valves, control valves, level controllers and the like would additionally be required in a commercial plant. The provision of such ancillary items of equipment forms no part of the present invention and would be in accordance with conventional chemical engineering practice. Moreover the various heat exchangers shown in Figures 2 and 3 are merely one means of obtaining the necessary temperature levels and heat fluxes. Other methods of effecting such heat exchange can of course be adopted in practice without departing from the scope of the present invention.

Referring to Figure 2 of the drawings, ethanol is supplied in line 101 to feed pump 102 and thence by way of line 103 to a vaporiser 104. This is heated by steam supplied in line 105. A minor amount of a refrigerated recycle stream containing ethanol and ethyl acetate, together with traces of iso-propanol and acetaldehyde, is also fed to vaporiser 104 in line 106.

A vaporous stream, which consists predominantly of ethanol, passes overhead in line 107 and is admixed with an azeotrope recycle stream in line 108. The combined stream then passes through

heat exchangers 109 and 110 and is admixed with hydrogen supplied from recycle compressor 111 in line 112.

The resulting vaporous mixture is supplied at a temperature of 273°C and at a pressure of 4.3 bar to reactor 113 which contains a charge of a catalyst which consists essentially of a reduced mixture of copper oxide and zinc oxide. The ethanol:$H_2$ molar ratio in this stream is approximately 300:1 and the liquid hourly space velocity is 0.35 $hr^{-1}$. There exits from reactor 113 in line 114 a mixture containing hydrogen, ethyl acetate, unconverted ethanol, a small amount of iso-propanol, and minor amounts of carbon oxides, methane, ethane and n-butanol. The ethanol:ethyl acetate molar ratio is approximately 2.4:1. This corresponds to a conversion of pure ethanol of 47.4% at a selectivity to ethyl acetate of 93.4%. This stream gives up heat to the incoming feed in heat exchanger 109, is cooled further in heat exchanger 115 and is condensed in condenser 116 against cooling water supplied in line 117.

The resulting condensate is separated from the gaseous components in knock-out pot 118 and is pumped through line 119 by pump 120 through heat exchanger 115 to product recovery column 121. This distillation column is heated by steam supplied in line 122 to its reboiler section and is operated at a pressure of 20.7 bar. Ethyl acetate product is recovered in line 123 whilst an approximately 20/80 mol/mol ethyl acetate/ethanol azeotrope is recovered overhead in line 124. Part of this is condensed in condenser 125 against cooling water supplied in line 126 and is recycled to the top of column 121 from condensate drum 127 in line 128 by pump 129. The remainder passes overhead from condensate drum 127 into line 108

through pressure let-down valve 130.

The overhead stream from knock-out pot 118 contains an appreciable amount of ethanol, ethyl acetate and acetaldehyde and passes through a demister pad 131 into line 132. A part of this overhead stream is fed in line 133 to the inlet end of recycle compressor 111. The remainder is fed to refrigeration unit 134, the condensate from which is fed by means of pump 135 to line 106. The gaseous components (mainly hydrogen together with minor amounts of carbon oxides, methane and ethane) are passed beyond battery limits in line 135' for use as fuel or for separation of $H_2$ in a suitable plant, for example a pressure swing absorption unit, in order to produce pure hydrogen.

Reference numeral 136 indicates the steam supply line for heat exchanger 110.

In Figure 3 the same reference numerals have been used as in Figure 2 to indicate like parts. Up to the product separation section the plant of Figure 3 is essentially the same as that of Figure 2.

Line 119 leads to an aldehyde separation column 140. Acetaldehyde appears in the overhead product in line 141 and is condensed in condenser 142 against cooling water supplied in line 143. A gas purge stream is taken in line 144 from aldehyde condensate drum 145. Part of the acetaldehyde condensate is returned to the top of column 140 in line 146, whilst the remainder is recycled in line 147 by means of pump 148 for admixture with the ethanol feed. Part of the bottoms product from column 140 is revaporised in reboiler 149 by steam supplied in line 150 and is returned to column 140 in line 151. The rest passes on in line 152 and then through throttle valve 153. In passage through valve 153 its pressure is reduced to 1 bar. The stream in line 152 is heated

in vaporiser 154 by steam supplied in line 155 and the resultant vaporous mixture is fed to a low pressure ethanol/azeotrope column 156 which yields almost pure ethanol as bottoms product. A major part of this ethanol bottoms product is recycled in line 157 by pump 158 and is admixed with the ethanol feed from line 103. The remainder of the bottoms product is revaporised by passage through reboiler 159 which is heated by steam supplied in line 160. The resultant vapour stream is fed back to the bottom of column 156 in line 161.

The overhead product from column 156 in line 162 is condensed in condenser 163 by means of cooling water supplied in line 164 and consists of a 53.8/46.2 mol ratio ethyl acetate/ethanol azeotrope. From the condensate drum 165 part is pumped back to the top of column 156 by pump 166, whilst the rest is fed in line 167 by pump 168, which raises its pressure again to 20.7 bar, to vaporiser 169; this is heated by steam supplied in line 170. The resultant vapour is fed to a high pressure ethanol/ethyl acetate azeotrope column 171. The top product of this column in line 172 is a 20/80 mol ratio ethyl acetate/ethanol azeotrope which is condensed in condenser 173 against cooling water supplied in line 174. Part of the resulting condensate from condensate drum 175 is recycled by pump 176 to the top of column 171. The remainder is fed in line 177 to throttle valve 178, which allows its pressure to drop again to 1 bar, for recycle to column 156.

The bottom product from column 171 is almost pure ethyl acetate which is recovered in line 179 and is passed through throttle valve 180 to cooler 181, in which it is cooled against cooling water in line 182, and thence to storage. Reference numeral 183

indicates the reboiler for column 171 and item 184 is its associated steam supply line.

CLAIMS

1. A process for the production of a primary $C_{2+}$ alkyl ester of an alkyl carboxylic acid which comprises contacting a vaporous mixture containing a primary $C_{2+}$ alkanol and hydrogen, said primary $C_{2+}$ alkanol preferably containing from 2 to about 8 carbon atoms, in an alkanol:hydrogen molar ratio of from about 1:10 to about 1000:1 at a combined partial pressure of alkanol and hydrogen of from about 0.1 bar up to about 40 bar and at a temperature in the range of from about 180°C to about 300°C in a catalytic reaction zone with a catalyst consisting essentially of a reduced mixture of copper oxide and zinc oxide, and recovering a reaction product mixture containing a primary $C_{2+}$ alkyl ester of an alkyl carboxylic acid, which ester contains twice as many carbon atoms as the primary $C_{2+}$ alkanol.

2. A process according to claim 1, in which the primary $C_{2+}$ alkanol is ethanol and the ester is ethyl acetate.

3. A process according to claim 1 or claim 2, in which the temperature is at least about 200°C, and preferably lies in the range of from about 230°C to about 280°C.

4. A process according to any one of claims 1 to 3, in which the combined partial pressure of primary alkanol and hydrogen lies in the range of from about 1 bar up to about 35 bar.

5. A process according to any one of claims 1 to 4, in which the catalyst consists essentially of a

reduced mixture of copper oxide and zinc oxide derived from a mixture comprising, before reduction, from about 10 to about 70 percent by weight CuO and about 90 to about 30 percent by weight ZnO, and preferably comprising from about 20 to about 40 percent by weight CuO and from about 60 to 80 percent by weight ZnO.

6. A process according to any one of claims 1 to 4, in which the catalyst consists essentially of a reduced mixture of copper oxide and zinc oxide derived from a mixture comprising, before reduction, from about 65 to about 85 percent by weight CuO and about 15 to about 35 percent by weight ZnO.

7. A process according to any one of claims 1 to 6, in which unreacted primary $C_{2+}$ alkanol is separated from at least the bulk of the product ester and is recycled to the catalytic reaction zone.

8. A process according to any one of claims 1 to 7, in which the reaction product mixture is distilled at elevated pressure, preferably at a pressure of from about 10 to about 40 bar, to yield an overhead product stream comprising an azeotrope of the primary $C_{2+}$ alkanol and said ester and a bottom product stream comprising predominantly said ester, and in which material of said overhead product stream is preferably recycled for admixture with fresh primary $C_{2+}$ alkanol feed.

9. A process according to claim 8, in which a hydrogen-containing purge stream is taken and is subjected to refrigeration for recovery of condensible components which are recycled for admixture with fresh primary $C_{2+}$ alkanol feed.

10. A process according to any one of claims 1 to 7, in which the reaction product mixture is distilled in a plurality of stages, including a low pressure distillation stage at a pressure of not more than about 5 bar and a subsequent high pressure distillation stage at a pressure of from about 10 bar to about 40 bar, said low pressure distillation stage resulting in production of a bottom product which comprises predominantly primary $C_{2+}$ alkanol and an overhead product which is a first azeotrope of the primary $C_{2+}$ alkanol and a major molar amount of said ester, the first azeotrope being thereafter subjected to distillation in said high pressure distillation stage to produce a bottom product which comprises predominantly said ester and an overhead product which is a second azeotrope of a major molar amount of the primary $C_{2+}$ alkanol and said ester, and in which said second azeotrope is preferably recycled to said low pressure distillation stage.

11. A process according to claim 10, in which the reaction product mixture is distilled essentially at reaction pressure, prior to said low pressure distillation stage, to remove alkanal by-product as an overhead product, and in which said alkanal by-product is preferably recycled for admixture with fresh primary $C_{2+}$ alkanol feed.

0151886

1/3

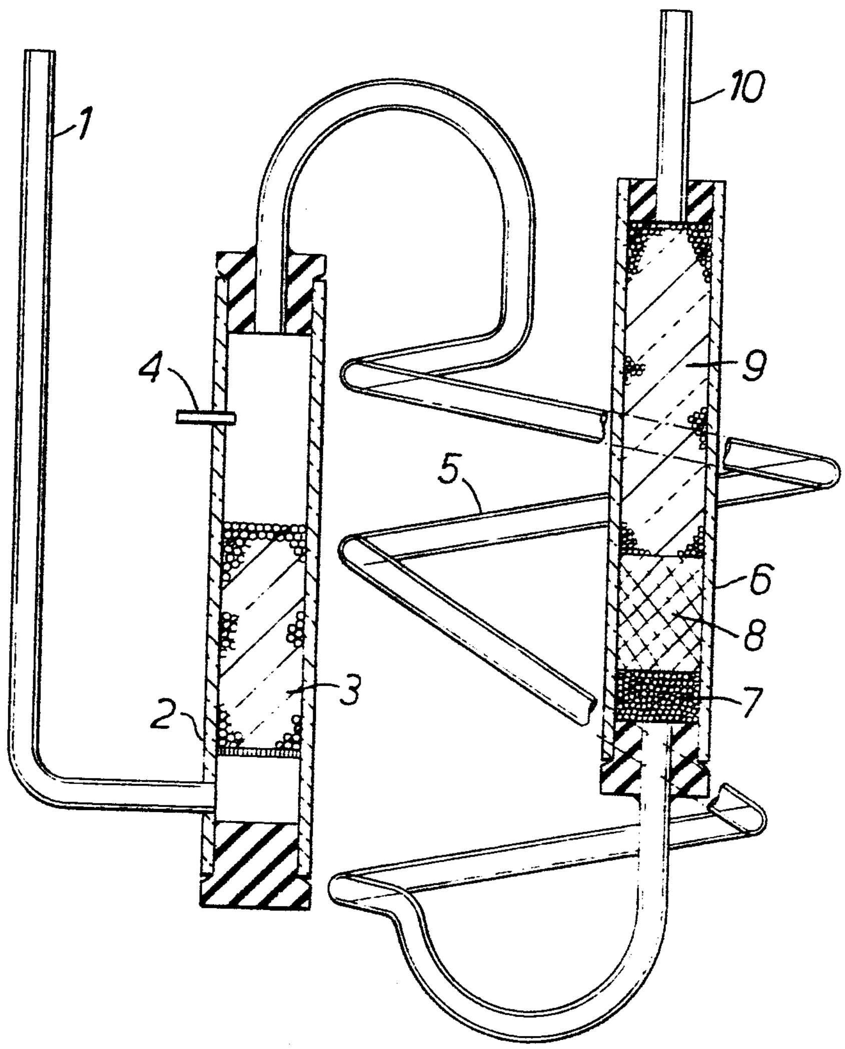


FIG.1.

0151886

2/3

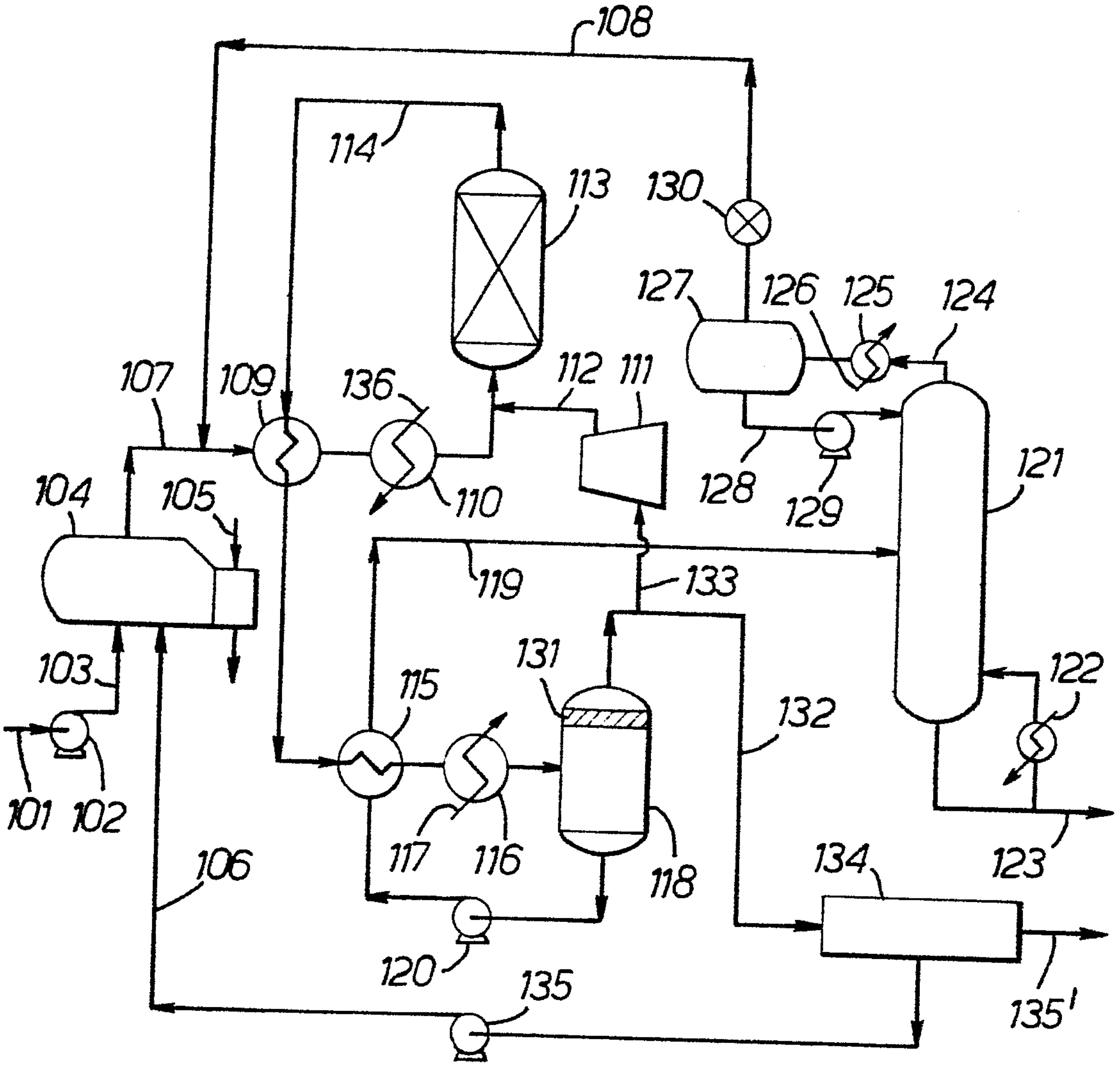


FIG. 2.

0151886

3/3

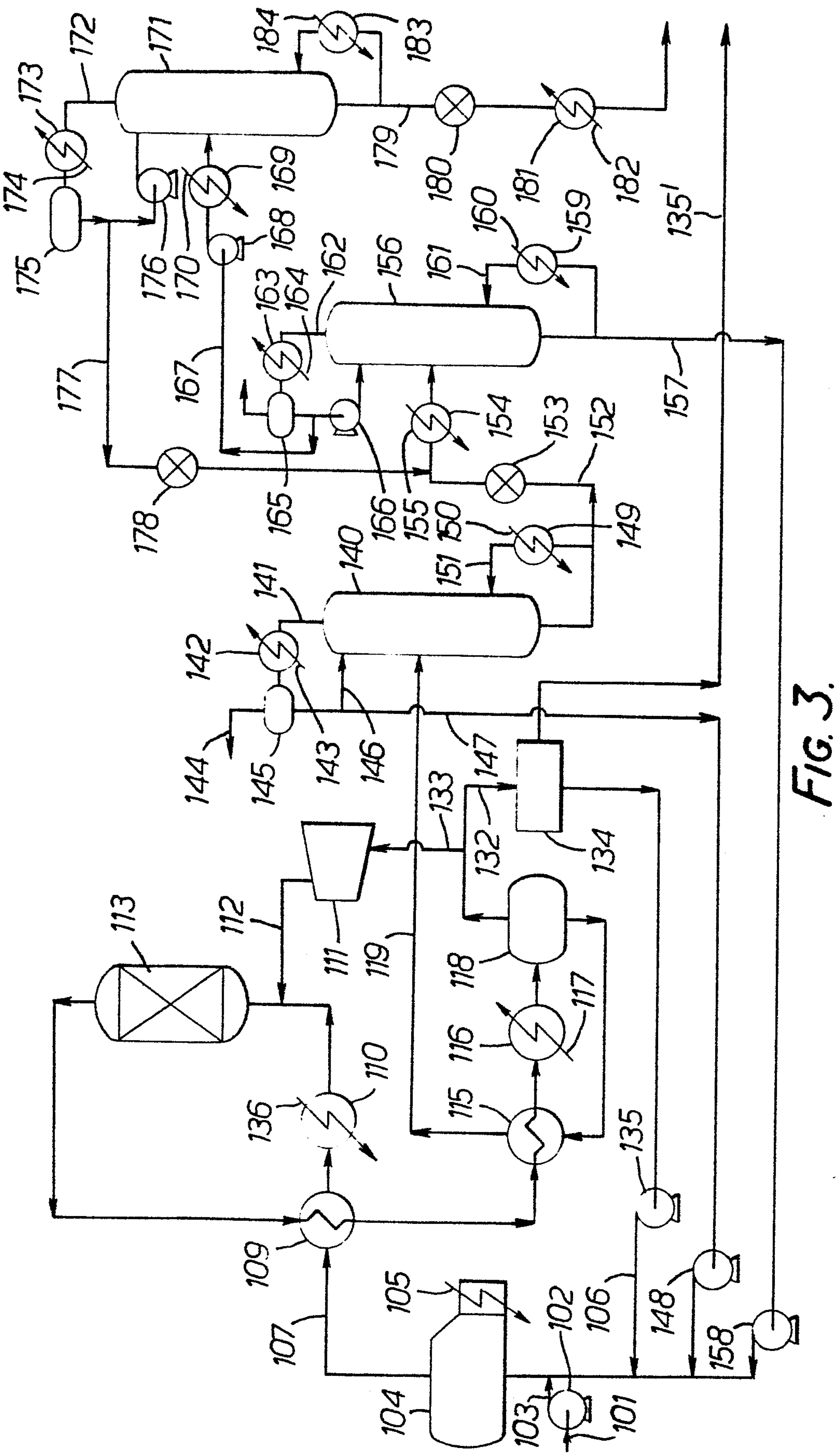


FIG. 3.

European Patent Office

# EUROPEAN SEARCH REPORT

0151886
Application number

EP 84309071.3

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl 4) |
| X | GB - A - 470 773 (USINES DE MELLE) * Page 2, line 23 - page 3, line 33 * | 1-4,7, 10 | C 07 C 69/14<br>C 07 C 67/40 |
| X | GB - A - 312 345 (E.I. DU PONT DE NEMOURS) * Page 2, line 24 - page 3, line 60 * | 1-4,6 | |
| X | GB - A - 287 846 (E.I. DU PONT DE NEMOURS) * Page 2, line 36 - page 3, line 58 * | 1-4 | |
| | | | TECHNICAL FIELDS SEARCHED (Int Cl 4) |
| | | | C 07 C 67/00<br>C 07 C 69/00 |

The present search report has been drawn up for all claims

| Place of search | Date of completion of the search | Examiner |
|---|---|---|
| VIENNA | 04-04-1985 | HOFBAUER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82